# EUROPEAN PATENT APPLICATION

(11) **EP 4 152 720 A1**
(43) Date of publication of application: **22.03.2023**
(21) Application number: 21804254.7
(22) Date of filing: 18.01.2021
(51) Int. Cl.: H04L 29/08, G16H 10/60, G16H 15/00, G06F 1/16

(54) **DATA TRANSMISSION METHOD AND ELECTRONIC DEVICE SUPPORTING SAME**

(30) Priority: 13.05.2020 KR 20200057283
(71) Applicant: Samsung Electronics Co., Ltd., Gyeonggi-do 16677 (KR)
(72) Inventor: LEE, Sungchull, Suwon-si Gyeonggi-do 16677 (KR); LEE, Jaemyung, Suwon-si Gyeonggi-do 16677 (KR); CHOI, Geunyoung, Suwon-si Gyeonggi-do 16677 (KR)
(74) Representative: Gulde & Partner
(86) International application number: PCT/KR2021/000676
(87) International publication number: WO 2021/230462

(57) **Abstract**

Disclosed is a wearable electronic device comprising at least one sensor, a communication circuit, and a processor functionally connected to the at least one sensor and the communication circuit, wherein the processor is configured to: receive, from an external electronic device connected via the communication circuit, a transmission request for first data satisfying an assigned condition, from among data measurable by using the at least one sensor, acquire the data measured by the at least one sensor, and transmit, to the external electronic device via the communication circuit, the first data satisfying the assigned condition, from among the acquired data. Various other embodiments as understood from the present document are also possible.

## Description

### [Technical Field]

Various embodiments of the present invention relate to data transmission techniques.

### [Background Art]

Recently, along with an increase of interest on health, the spread of electronic devices for providing health care services is increasing. These electronic devices can acquire and store exercise information, medical information, meal information, or sleep information as well as user's biometric data measured through a sensor, and can determine a user's health state by analyzing the acquired information. Also, the electronic device can provide a user interface for displaying and managing information on the user's health state.

On the other hand, as the spread of wearable electronic devices worn on or attached directly to the user's body is increasing, the electronic device can be communicatively connected to the wearable electronic device equipped with a sensor and receive data measured through the sensor from the wearable electronic device. Also, the electronic device can analyze the data received from the wearable electronic device and determine the user's health state, and display and manage information on the determined health state.

### [Disclosure of Invention]

### [Technical Problem]

However, the existing electronic device can receive a large amount of measured data from the wearable electronic device in order to determine the user's health state or to synchronize the data. In this case, even if various methods such as encoding or compression are used, there is no choice but to be a limit in reducing the size of received data. Also, when data for data synchronization is received, since the existing electronic device receives all data within a specified time range in consideration of a synchronization period from the wearable electronic device, it can be difficult to selectively or variably receive only data required by the electronic device.

Various embodiments of the present invention may present a method of selectively or variably transmitting only data satisfying a specified condition required by an electronic device, and an electronic device supporting the same.

Also, various embodiments of the present invention may present a method of continuously transmitting data within one session, and an electronic device supporting the same.

Also, various embodiments of the present invention may present a method for selectively or variably transmitting measured data, based on data acquired from an external electronic device, and an electronic device supporting the same.

### [Solution to Problem]

A wearable electronic device of various embodiments of the present invention may include at least one sensor, a communication circuit, and a processor functionally connected to the at least one sensor and the communication circuit. The processor may be configured to receive a request for transmission of first data satisfying a specified condition among data that is measurable using the at least one sensor, from an external electronic device connected via the communication circuit, acquire the data measured through the at least one sensor, and transmit the first data satisfying the specified condition among the acquired data, to the external electronic device via the communication circuit.

Also, an electronic device of various embodiments of the present invention may include a communication circuit, a display, a memory, and a processor functionally connected to the communication circuit, the display and the memory. The processor may be configured to receive information on data measurable through at least one sensor included in a wearable electronic device, from the wearable electronic device connected via the communication circuit, and transmit a request for transmission of first data satisfying a specified condition among the data, to the wearable electronic device via the communication circuit, based on the received information, and receive the first data from the wearable electronic device via the communication circuit, and display, through the display, at least one first object that is provided based on the received first data.

Also, a data transmission method of various embodiments of the present invention may include an operation of receiving a request for transmission of first data satisfying a specified condition among data measurable using at least one sensor, from an external electronic device connected via a communication circuit, an operation of acquiring the data measured through the at least one sensor, and an operation of transmitting the first data satisfying the specified condition among the acquired data, to the external electronic device via the communication circuit.

### [Advantageous Effects of Invention]

According to various embodiments of the present invention, resource consumption required for data transmission and processing may be reduced by selectively or variably transmitting only data satisfying a specified condition.

Also, according to various embodiments of the present invention, by continuously transmitting data within one session, a request for overlapped data may be omitted and thus the consumption of network resources may be reduced.

Also, according to various embodiments of the present invention, the accuracy and reliability of transmitted data may be improved by selectively or variably transmitting measured data, based on data acquired from an external electronic device.

In addition, various effects directly or indirectly identified through the present document may be presented.

### [Brief Description of Drawings]

FIG 1 is a block diagram of an electronic device in a network environment according to various embodiments.
FIG 2 is a diagram for explaining a construction of a wearable electronic device and an electronic device according to an embodiment of the present invention.
FIG 3 is a diagram for explaining a method of operating a wearable electronic device related to data transmission according to an embodiment of the present invention.
FIG 4 is a diagram for explaining a method of operating an electronic device related to data reception according to an embodiment of the present invention.
FIG 5 is a diagram for explaining a method of transmitting data through a session connection between a wearable electronic device and an electronic device according to an embodiment of the present invention.
FIG 6 is a diagram for explaining another method of transmitting data through a session connection between a wearable electronic device and an electronic device according to an embodiment of the present invention.
FIG 7 is a diagram for explaining a method of acquiring necessary data according to the type of data according to an embodiment of the present invention.
FIG 8 is a diagram for explaining a process of data transmission between a wearable electronic device and an electronic device according to an embodiment of the present invention.
FIG 9 is a diagram for explaining a process of data transmission through a session connection between a wearable electronic device and an electronic device according to an embodiment of the present invention.
FIG 10 is a diagram for explaining another process of data transmission through a session connection between a wearable electronic device and an electronic device according to an embodiment of the present invention.
FIG 11 is a diagram for explaining a process of acquiring necessary data according to the type of data according to an embodiment of the present invention.
FIG 12 is a diagram for explaining another process of acquiring necessary data according to the type of data according to an embodiment of the present invention.
FIG 13 is a diagram for explaining a process of data processing request according to an embodiment of the present invention.

In connection with a description of the drawings, the same or similar reference numerals may be used for the same or similar components.

### [Mode for Carrying out the Invention]

Hereinafter, various embodiments of the present invention will be described with reference to the accompanying drawings. For convenience of description, the size of the components shown in the drawings may be exaggerated or reduced, and the present invention is not necessarily limited as illustrated.

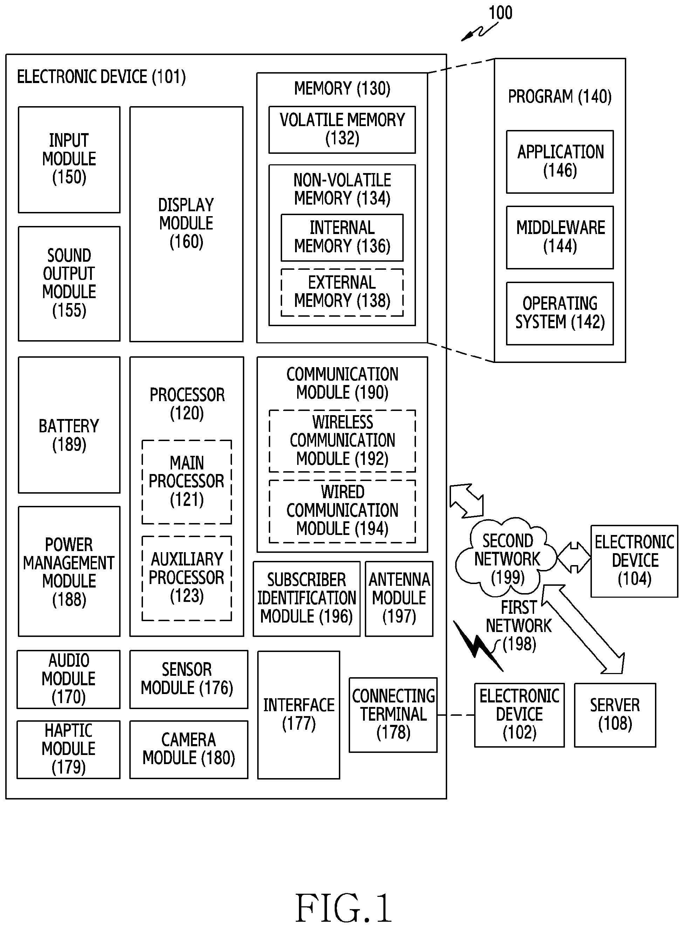

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mm Wave antenna module. According to an embodiment, the mm Wave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

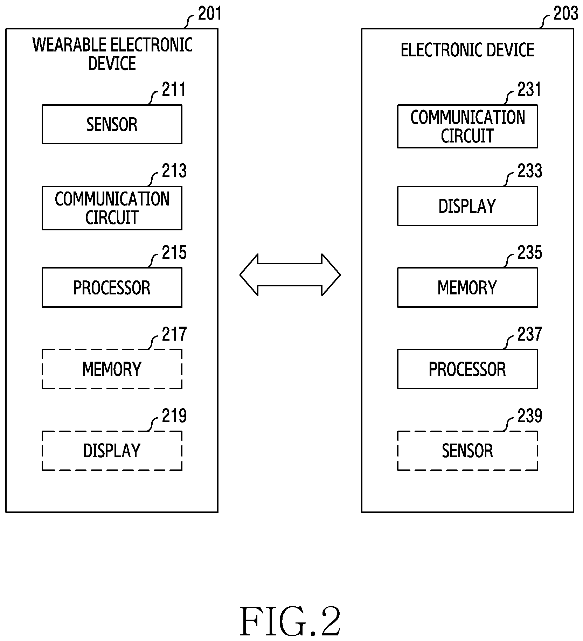

FIG 2 is a diagram for explaining a construction of a wearable electronic device and an electronic device according to an embodiment of the present invention.

Referring to FIG 2, the electronic device 203 may be communication connected with the wearable electronic device 201 equipped with a sensor 211 and receive data measured through the sensor 211 from the wearable electronic device 201. Also, the electronic device 203 may analyze data received from the wearable electronic device 201 and determine a user's health state, and display and manage information on the determined health state. In this case, the electronic device 203 does not receive all the measured data from the wearable electronic device 201, but may transmit a data transmission request satisfying a specified condition to the wearable electronic device 201 in order to receive only necessary data, and the wearable electronic device 201 receiving the transmission request may selectively or variably transmit only the data satisfying the specified condition among the data measured through the sensor 211 to the electronic device 203.

The wearable electronic device 201 may include the sensor 211 (e.g., the sensor module 176 of FIG 1), a communication circuit 213 (e.g., the communication module 190 of FIG 1), and a processor 215 (e.g., the processor 120 of FIG 1). However, a construction of the wearable electronic device 201 is not limited thereto. According to various embodiments, the wearable electronic device 201 may further include at least one other component in addition to the above-described components. According to an embodiment, the wearable electronic device 201may further include at least one of a memory 217 (e.g., the memory 130 of FIG 1) for storing at least one of data measured through the sensor 211 and data received from an external electronic device (e.g., the electronic device 203) via the communication circuit 213, and a display 219 (e.g., the display module 160 of FIG 1) for displaying information on a user's health state determined using at least one of the data measured through the sensor 211 and the data received from the external electronic device (e.g., the electronic device 203) via the communication circuit 213.

The sensor 211 may measure or detect a user's biological signal by contacting or coming close to a part of the user's body. For example, the sensor 211 may include at least one of a photoplethysmography (PPG) sensor and an electrocardiogram (ECG) sensor. The PPG sensor may include a light emitting unit (e.g., an LED or a laser diode) for irradiating light and a light receiving unit (e.g., a photodiode or an image sensor) for detecting light reflected from the user's skin or transmitted through the skin among the irradiated light, and measure at least one of user's heart rate, blood pressure, blood sugar, blood volume, and oxygen saturation by converting light detected through the light receiving unit into an electrical signal and processing the converted electrical signal. The ECG sensor may include a plurality of electrodes, and may measure a user's electrocardiogram (ECG) by measuring a potential change dependent on the activity of a myocardial cell through the plurality of electrodes that come in contact with the user's skin. The type of the sensor 211 capable of measuring or detecting the user's biological signal is not limited thereto. The sensor 211 may include, in addition to the PPG sensor or the ECG sensor, various sensors capable of measuring or detecting the user's biological signal by contacting or coming close to a part of the user's body.

The sensor 211 may measure or detect the movement of the wearable electronic device 201 worn on a part of the user's body. For example, the sensor 211 may include at least one of a motion sensor and a pedometer. The motion sensor may include at least one of an acceleration sensor, a gyro sensor, and a geomagnetic sensor. In a state in which the wearable electronic device 201 is worn on a part of the user's body, the wearable electronic device 201 may move according to the user's movement, and the motion sensor may detect the movement of the wearable electronic device 201. Also, the processor 215, which acquires sensing data related to the movement of the wearable electronic device 201 from the motion sensor, may determine a user's movement state (e.g., the number of steps) or the degree of movement during user's sleep and a sleeping posture through the movement of the wearable electronic device 201. The pedometer may measure the number of steps of the user by detecting the movement of the wearable electronic device 201.

According to an embodiment, the wearable electronic device 201 may include a plurality of sensors 211. In this case, the wearable electronic device 201 may select at least one of the plurality of sensors 211, and measure or detect data related to at least one of user's biometric information and activity information. The user's biometric information may include, for example, at least one of user's heart rate, blood pressure, blood sugar, blood volume, oxygen saturation, and electrocardiogram. The user's activity information may include, for example, at least one of information (e.g., the number of steps) on an exercise state such as running or walking, or information on a sleep state such as the degree of movement during sleep or a sleeping posture.

The communication circuit 213 may support communication between the wearable electronic device 201 and an external electronic device (e.g., the electronic device 203). For example, the wearable electronic device 201 may transmit and/or receive commands or data to and from the electronic device 203 via the communication circuit 213. According to an embodiment, the communication circuit 213 may transmit and/or receive commands or data to and from the electronic device 203 through a communication method such as Bluetooth or WiFi direct.

The processor 215 may control at least one other component of the wearable electronic device 201, and may perform various data processing or operations. For example, the processor 215 may execute a command related to transmission of data measured through the sensor 211.

The processor 215 may receive a data transmission request satisfying a specified condition from an external electronic device (e.g., the electronic device 203) via the communication circuit 213. The specified condition may include, for example, the type of the sensor 211 used for measurement, the type of data measured through the selected sensor 211, and a measurement period. The measurement period may indicate a time for measuring data through the sensor 211. In an example, when the measurement period includes a previous time range based on a current time point, the processor 215 may acquire data corresponding to the measurement period among data already measured through the sensor 211. In this case, the processor 215 may extract the data corresponding to the measurement period from the memory 217. In another example, when the measurement period includes a current time point or includes a subsequent time range based on the current time point, the processor 215 may measure data during the measurement period through the sensor 211. Or, the processor 215 may measure data during a period including the measurement period through the sensor 211, and selectively acquire only data corresponding to the measurement period among the measured data. According to an embodiment, the measurement period may include a continuous time range from a start time to an end time. In another embodiment, the measurement period may include a plurality of time ranges in which different consecutive time ranges are spaced apart for at least a predetermined time. For example, the measurement period may include a plurality of periodic time ranges.

The processor 215 may acquire data (sensing data) through the sensor 211. For example, the processor 215 may acquire data related to at least one of user's biometric information and activity information through the sensor 211. According to an embodiment, when the wearable electronic device 201 includes the plurality of sensors 211, the processor 215 may select at least one sensor 211 for acquiring data satisfying the specified condition from among the plurality of sensors 211. For example, the processor 215 may select at least one sensor 211 from among the plurality of sensors 211, based on the type of the sensor 211 included in the specified condition. Also, the processor 215 may activate the selected at least one sensor 211, and acquire data related to at least one of the user's biometric information and activity information through the activated at least one sensor 211.

The processor 215 may select data satisfying the specified condition from among the data acquired through the sensor 211. For example, the processor 215 may select data corresponding to the type of the sensor 211, the type of data measured through the selected sensor 211, and a measurement period included in the specified condition, from among the acquired data. According to an embodiment, the processor 215 may select data corresponding to the type of data and the measurement period included in the specified condition, from among the data measured through the at least one activated sensor 211.

The processor 215 may transmit data satisfying the specified condition among data acquired through the sensor 211, to the external electronic device (e.g., the electronic device 203) via the communication circuit 213. For example, the processor 215 may transmit data selected among the data measured through the activated at least one sensor 211, to the external electronic device via the communication circuit 213. According to an embodiment, after transmitting the selected data to the external electronic device, the processor 215 may deactivate the activated at least one sensor 211.

According to an embodiment, the processor 215 may transmit a request for transmission of second data related to first data, to the external electronic device (e.g., the electronic device 203) via the communication circuit 213. The first data may be data related to data requested to be transmitted from the external electronic device. The second data may be data used to measure or detect the first data. In an example, when the first data is data indicating a user's calorie consumption amount, the second data may be data indicating at least one of user's gender, height, weight, stride length, or an altitude value of a user's current location. In another example, when the first data is data indicating a user's sleep state or stress state, the second data may be data indicating a user's caffeine intake amount or exercise amount before sleep. In a further example, when the first data is data indicating a heart disease state being based on a user's blood sugar or electrocardiogram, the second data may be data indicating user's food intake information.

According to an embodiment, the processor 215 may store the second data in the memory 217. According to another embodiment, the processor 215 does not store the second data in the memory 217 but, when the second data is needed for measurement or detection of the first data, the processor 215 may transmit a request for transmission of the second data to the external electronic device (e.g., the electronic device 203) via the communication circuit 213 and receive the second data from the external electronic device.

According to an embodiment, when the processor 215 receives the second data related to the first data from the external electronic device (e.g., the electronic device 203) via the communication circuit 213, the processor 215 may correct the first data, based on the second data. In an example, the processor 215 may correct first data indicating a user's calorie consumption amount, based on second data indicating at least one of a user's gender, height, weight, stride length, or an altitude value of a user's current location. In another example, the processor 215 may correct first data indicating a user's sleep state or stress state, based on the second data indicating a user's caffeine intake amount or exercise amount before sleep. In a further example, the processor 215 may correct first data indicating a heart disease state being based on a user's blood sugar or electrocardiogram, based on second data indicating user's food intake information. Accordingly, since the processor 215 may more accurately reflect a user's physical condition or user's current state at the time of measuring or detecting the first data, the accuracy and reliability of the first data may be improved.

According to an embodiment, the processor 215 may transmit a request for processing of data measured through the sensor 211, to the external electronic device (e.g., the electronic device 203) via the communication circuit 213. For example, the processor 215 may request the external electronic device to calculate or analyze data, based on the measured data. In an example, when the processor 215 may measure a heart rate through the sensor 211 but has no analysis engine capable of analyzing a heart rate variability (HRV) or arrhythmia, the processor 215 may request the analysis of the heart rate variability or arrhythmia while transmitting data on the measured heart rate to the external electronic device via the communication circuit 213. In another example, when the processor 215 may measure data indicating a sleep state through the sensor 211 but has no analysis engine for four stages of sleep (e.g., REM sleep (REM) and three stages of non-REM sleep (NREM)), the processor 215 may request the analysis of the four stages of sleep while transmitting the measured data indicating the sleep state to the external electronic device via the communication circuit 213.

The electronic device 203 may include a communication circuit 231 (e.g., the communication module 190 of FIG 1), a display 233 (e.g., the display module 160 of FIG 1), a memory 235 (e.g., the memory 130 of FIG 1), and a processor 237 (e.g., the processor 120 of FIG 1). However, a construction of the electronic device 203 is not limited thereto. According to various embodiments, the electronic device 203 may further include at least one other component in addition to the above-described components. According to an embodiment, the electronic device 203 may further include a sensor 239 (e.g., the sensor module 176 of FIG 1) capable of coming in contact with or coming close to a part of the user's body and measuring or detecting a user's biological signal, or measuring or detecting the movement of the electronic device 203. The sensor 239 may include, for example, an altitude measurement sensor capable of measuring an altitude value of a user's current location.

The communication circuit 231 may support communication between the electronic device 203 and an external electronic device (e.g., the wearable electronic device 201). For example, the electronic device 203 may transmit and/or receive commands or data to and from the wearable electronic device 201 via the communication circuit 231.

The display 233 may display various contents (e.g., a text, an image, a video, an icon or a symbol) to a user. In an example, the display 233 may display at least one of data received from the external electronic device (e.g., the wearable electronic device 201) via the communication circuit 231 or information (e.g., information on a user's health condition) processed and analyzed by the processor 237 based on the received data. According to an embodiment, the display 233 may include a touch screen. For example, the display 233 may receive a touch, gesture, proximity, or hovering input that uses an electronic pen or a part of the user's body.

The memory 235 may store various data used by at least one component of the electronic device 203. For example, the memory 235 may store at least one of data received from the external electronic device (e.g., the wearable electronic device 201) via the communication circuit 231 or information (e.g., information on a user's health condition) processed or analyzed by the processor 237 based on the received data. According to an embodiment, at least one of the data or information stored in the memory 235 may be stored and managed in the form of a table.

The processor 237 may control at least one other component of the electronic device 203, and may perform various data processing or operations. For example, the processor 237 may execute an instruction related to transmission and/or reception of data via the communication circuit 231 stored in the memory 235.

The processor 237 may request the transmission of data satisfying a specified condition to an external electronic device (e.g., the wearable electronic device 201) via the communication circuit 231. The specified condition may include, for example, the type of the sensor 211 used for measurement among the plurality of sensors 211 included in the external electronic device, the type of data measured through the selected sensor 211, and a measurement period.

According to an embodiment, before the data transmission request, the processor 237 may receive information on data measurable through the sensor 211 included in the external electronic device, from the external electronic device (e.g., the wearable electronic device 201) via the communication circuit 231. For example, the processor 237 may receive information on the type of the sensor 211 included in the external electronic device and the type of data measurable through the sensor 211 via the communication circuit 231. In this case, the processor 237 may set the specified condition, based on information on the type of the sensor 211 included in the external electronic device and the type of data measurable through the sensor 211. According to an embodiment, the processor 237 may display information on the type of the sensor 211 included in the external electronic device and the type of data measurable through the sensor 211 through the display 233. In this case, the processor 237 may set the specified condition, based on the information displayed on the display 233 and a user's input. For example, the processor 237 may display information on the type of the sensor 211 included in the external electronic device and the type of data measurable through the sensor 211 on the display 233 in the form of a list, and may set the specified condition, based on a user's input for selecting at least one of items included in the list. Here, each of the items included in the list may include an object to which the information on the type of the sensor 211 included in the external electronic device, the type of data measurable through the sensor 211, and the measurement period is mapped.

The processor 237 may receive data satisfying the specified condition from the external electronic device (e.g., the wearable electronic device 201) via the communication circuit 231. The data satisfying the specified condition may include data that is selected based on the type of data and the measurement period included in the specified condition, among the data measured through at least one sensor 211 that is selected based on the type of the sensor 211 included in the specified condition among the plurality of sensors 211 included in the external electronic device.

The processor 237 may provide at least one object, based on data received from the external electronic device (e.g., the wearable electronic device 201) via the communication circuit 231, and may display the provided at least one object through the display 233. The at least one object may include, for example, information related to a user's health state. The information related to the user's health state may include, for example, at least one of user's biometric information, user's activity information, or health information determined based on the user's biometric information and activity information. The at least one object may include at least one of a text and an image. In some embodiment, the processor 237 may output voice information corresponding to the at least one object through a speaker (e.g., the sound output module 155 of FIG 1) as well.

According to an embodiment, the processor 237 may receive a request for transmission of second data related to first data from the external electronic device (e.g., the wearable electronic device 201) via the communication circuit 231. The first data may be data related to data requested to be transmitted from the electronic device 203. The second data may be data used to measure or detect the first data. In an example, when the first data is data indicating a user's calorie consumption amount, the second data may be data indicating at least one of a user's gender, height, weight, stride length, or an altitude value of a user's current location. The processor 237 may extract at least one of the user's gender, height, and weight from user's profile information stored in the memory 235. Also, the processor 237 may calculate a user's stride length, based on the user's height stored in the memory 235. Also, the processor 237 may measure the altitude value of the user's current location through the sensor 239. In another example, when the first data is data indicating a user's sleep state or stress state, the second data may be data indicating the user's caffeine intake amount or exercise amount before sleep. The processor 237 may acquire the user's caffeine intake amount before sleep, based on a user input. Also, the processor 237 may measure the user's exercise amount through the sensor 239. In another example, when the first data is data indicating a heart disease state based on a user's blood sugar or electrocardiogram, the second data may be data indicating user's food intake information. The processor 237 may acquire the user's food intake information, based on the user input.

According to an embodiment, the processor 237 may receive a user input through the touch screen display 233. According to another embodiment, the electronic device 203 may further include an input device (e.g., the input module 150 of FIG 1) for receiving a user input, and the processor 237 may receive the user input through the input device.

According to an embodiment, the processor 237 may receive a data processing request from the external electronic device (e.g., the wearable electronic device 201) via the communication circuit 231. For example, the processor 237 may be requested to calculate or analyze, together with data measured through the sensor 211 of the external electronic device, data being based on the measured data. In an example, the processor 237 may be requested to analyze a heart rate variability or arrhythmia while receiving data on a measured heart rate from the external electronic device via the communication circuit 231. In another example, the processor 237 may be requested to analyze four stages of sleep while receiving data indicating a measured sleep state from the external electronic device via the communication circuit 231. In this case, the processor 237 may calculate or analyze data, based on data received from the external electronic device, and may transmit the calculated or analyzed data to the external electronic device via the communication circuit 231.

According to an embodiment, the wearable electronic device 201 and the electronic device 203 may transmit and/or receive data through a session. In a communication connection between devices (e.g., the wearable electronic device 201 and the electronic device 203) via respective communication circuits (e.g., the communication circuit 213 and the communication circuit 231), the session may indicate a logical connection including a period from the start to the end of communication for data transmission and/or reception. When the session is established, the wearable electronic device 201 and the electronic device 203 may transmit and/or receive data satisfying a specified condition while the established session is maintained. Since one session requires only one data transmission request, a plurality of data satisfying a specified condition may be transmitted and/or received while the one session is maintained. The established session may be ended when the transmission and/or reception of data satisfying the specified condition is completed. Also, when a plurality of data satisfying different conditions are transmitted and/or received between the wearable electronic device 201 and the electronic device 203, a plurality of different sessions may be established to transmit and/or receive data satisfying each condition. For example, the wearable electronic device 201 and the electronic device 203 may transmit and/or receive data satisfying a first specified condition through a first session, and may transmit and/or receive data satisfying a second specified condition through a second session.

As described above, according to various embodiments, a wearable electronic device (e.g., the wearable electronic device 201 of FIG 2) may include at least one sensor (e.g., the sensor 211 of FIG 2), a communication circuit (e.g., the communication circuit 213 of FIG 2), and a processor (e.g., the processor 215 of FIG 2) functionally connected to the at least one sensor and the communication circuit. The processor may be configured to receive a request for transmission of first data satisfying a specified condition among data that is measurable using the at least one sensor, from an external electronic device (e.g., the electronic device 203 of FIG 2) connected via the communication circuit, acquire the data measured through the at least one sensor, and transmit the first data satisfying the specified condition among the acquired data, to the external electronic device via the communication circuit.

According to various embodiments, the processor may be configured to establish a first session for transmission of the first data, in response to receiving the request for transmission of the first data.

According to various embodiments, the processor may be configured to establish a second session for transmission of second data, when receiving a request for transmission of the second data satisfying a condition different from the specified condition among the data from the external electronic device via the communication circuit.

According to various embodiments, the processor may be configured to transmit information on the data that is measurable using the at least one sensor, to the external electronic device via the communication circuit.

According to various embodiments, the processor may be configured to transmit a request for transmission of second data related to the first data, to the external electronic device via the communication circuit, and when receiving the second data from the external electronic device via the communication circuit, correcting the first data, based on the received second data, and transmit the corrected first data to the external electronic device via the communication circuit.

According to various embodiments, the processor may be configured to transmit a plurality of second data measured by the at least one sensor and a request for processing of the plurality of second data, to the external electronic device via the communication circuit, and receive third data that is calculated by processing the plurality of second data, from the external electronic device via the communication circuit.

As described above, according to various embodiments, an electronic device (e.g., the electronic device 203 of FIG 2) may include a communication circuit (e.g., the communication circuit 231 of FIG 2), a display (e.g., the display 231 of FIG 2), a memory (e.g., the memory 235 of FIG 2), and a processor (e.g., the processor 237 of FIG 2) functionally connected to the communication circuit, the display and the memory. The processor may be configured to receive information on data measurable through at least one sensor (e.g., the sensor 211 of FIG 2) included in a wearable electronic device (e.g., the wearable electronic device 201 of FIG 2), from the wearable electronic device connected via the communication circuit, and transmit a request for transmission of first data satisfying a specified condition among the data, to the wearable electronic device via the communication circuit, based on the received information, and receive the first data from the wearable electronic device via the communication circuit, and display, through the display, at least one first object that is provided based on the received first data.

According to various embodiments, the processor may be configured to establish a first session for requesting the transmission of the first data and receiving the first data.

According to various embodiments, the processor may be configured to establish a second session for requesting the transmission of second data satisfying a condition different from the specified condition among the data and receiving the second data.

According to various embodiments, the processor may be configured to display, through the display, at least one second object that is provided based on the received information, and receive a user input for selecting the at least one second object, and set the specified condition, based on the received user input.

According to various embodiments, the at least one second object may include an object to which information on at least one of the type of the at least one sensor included in the wearable electronic device, the type of the data measurable through the at least one sensor, and a measurement period is mapped.

According to various embodiments, the processor may be configured to, when receiving a request for transmission of second data related to the first data from the wearable electronic device via the communication circuit, check the type of the second data, and acquire the second data by using at least one sensor (e.g., the sensor 239 of FIG 2) included in the electronic device or acquire the second data through extraction from the memory, in accordance with the checked type of the second data, and transmit the acquired second data to the wearable electronic device via the communication circuit.

According to various embodiments, the processor may be configured to extract a first portion of the second data from a first table that is stored in the memory, and extract a second portion of the second data from a second table that is stored in the memory.

According to various embodiments, the electronic device may be configured to merge the extracted first portion of the second data and the extracted second portion of the second data and transmit the merged portion to the wearable electronic device via the communication circuit.

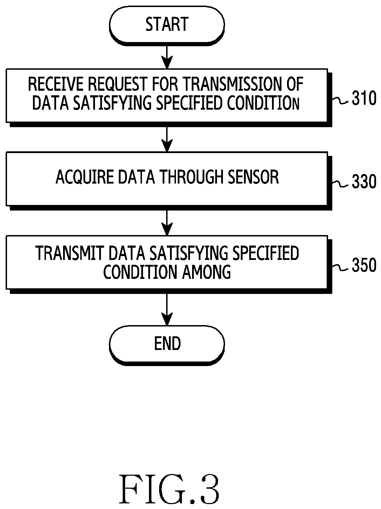

FIG 3 is a diagram for explaining a method of operating a wearable electronic device related to data transmission according to an embodiment of the present invention.

Referring to FIG 3, in operation 310, a processor (e.g., the processor 215 of FIG 2) of the wearable electronic device (e.g., the wearable electronic device 201 of FIG 2) may receive a data transmission request satisfying a specified condition from an external electronic device (e.g., the electronic device 203 of FIG 2) via a communication circuit (e.g., the communication circuit 213 of FIG 2). The specified condition may include, for example, the type of a sensor (e.g., the sensor 211 of FIG 2) used for measurement included in the wearable electronic device, the type of data measured through the selected sensor, and a measurement period. When a plurality of sensors (e.g., the sensors 211 of FIG 2) are included in the wearable electronic device, the specified condition may include the type of at least one sensor used for measurement among the plurality of sensors, the type of data measured through the selected at least one sensor, and the measurement period. The measurement period may indicate a time for measuring data through the selected at least one sensor. According to an embodiment, the measurement period may include a continuous time range from a start time to an end time. In another embodiment, the measurement period may include a plurality of time ranges in which different consecutive time ranges are spaced apart for at least a predetermined time. For example, the measurement period may include a plurality of periodic time ranges.

In operation 330, the processor may acquire data (sensing data) through the sensor. For example, the processor may acquire data related to at least one of user's biometric information and activity information through the sensor. According to an embodiment, when the data requested to be transmitted includes data previously already measured through the sensor with a criterion of a current time point, the processor may not perform operation 330. In this case, the processor may use data that is already measured through the sensor and is stored in a memory (e.g., the memory 217 of FIG 2). In this regard, the processor may determine whether the data requested to be transmitted includes the data previously already measured by the sensor with a criterion of the current time point, based on at least one of the type of the sensor, the type of the data, and the measurement period.

According to an embodiment, when the wearable electronic device includes a plurality of sensors, the processor may select at least one sensor among the plurality of sensors, based on the type of a sensor included in the specified condition. Also, the processor may activate the selected at least one sensor, and acquire data related to at least one of user's biometric information and activity information through the activated at least one sensor.

When data is acquired through the sensor, the processor may select data satisfying the specified condition, from among the data acquired through the sensor. For example, the processor may select data corresponding to the type of data and a measurement period included in the specified condition, from among the data acquired through the sensor.

According to an embodiment, when the measurement period includes a previous time range being based on a current time point, the processor may acquire data corresponding to the measurement period, from among data already measured through the sensor. In this case, the processor may extract data corresponding to the measurement period from the memory. For another example, when the measurement period includes the current time point or includes a subsequent time range being based on the current time point, the processor may measure data during the measurement period through the sensor. Or, the processor may measure data during a period including the measurement period through the sensor, and selectively acquire only data corresponding to the measurement period from among the measured data as well.

In operation 350, the processor may transmit the selected data to the external electronic device via the communication circuit. For example, the processor may transmit data satisfying the specified condition among the acquired data, to the external electronic device via the communication circuit.

According to an embodiment, the processor may deactivate the at least one activated sensor, after transmitting the selected data to the external electronic device.

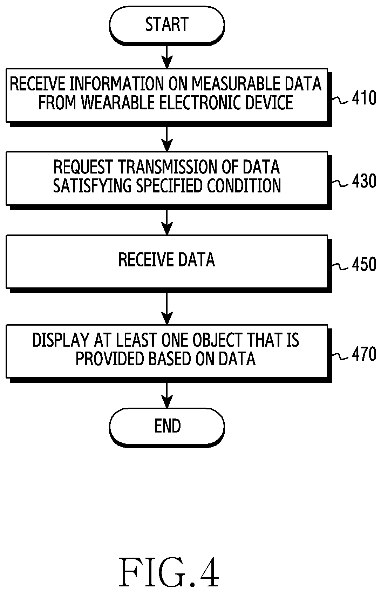

FIG 4 is a diagram for explaining a method of operating an electronic device related to data reception according to an embodiment of the present invention.

Referring to FIG 4, in operation 410, a processor (e.g., the processor 237 of FIG 2) of the electronic device (e.g., the electronic device 203 of FIG 2) may receive information on data measurable through a sensor (e.g., the sensor 211 of FIG 2) included in an external electronic device, from the external electronic device (e.g., the wearable electronic device 201 of FIG 2) via a communication circuit (e.g., the communication circuit 231 of FIG 2). For example, the processor may receive information on the type of a sensor included in the external electronic device and the type of data measurable through the sensor, via the communication circuit.

When receiving the information on the type of the sensor included in the external electronic device and the type of data measurable through the sensor, the processor may set a specified condition, based on the information on the type of the sensor included in the external electronic device and the type of data measurable through the sensor. For example, the processor may set the specified condition regarding the data measurable through the sensor of the external electronic device in order to display information related to a user's health state. The specified condition may include, for example, the type of a sensor (e.g., the sensor 211 of FIG 2) used for measurement included in the external electronic device, the type of data measured through the selected sensor, and a measurement period. When the external electronic device includes a plurality of sensors (e.g., the sensors 211 of FIG 2), the specified condition may include the type of at least one sensor used for measurement among the plurality of sensors, the type of data measured through the selected at least one sensor, and the measurement period.

According to an embodiment, the processor may display, through a display (e.g., the display 233 of FIG 2), information on the type of a sensor included in the external electronic device and the type of data measurable through the sensor. In this case, the processor may set the specified condition, based on the information displayed on the display and a user's input. For example, the processor may display information on the type of the sensor included in the external electronic device and the type of data measurable through the sensor, on the display, in the form of a list, and may set the specified condition, based on a user input for selecting at least one of items included in the list. Here, each of the items included in the list may include an object to which information on the type of the sensor included in the external electronic device, the type of data measurable through the sensor, and the measurement period is mapped.

In operation 430, the processor may request the transmission of data satisfying the specified condition, to the external electronic device via the communication circuit. Also, in operation 450, the processor may receive the data satisfying the specified condition from the external electronic device via the communication circuit. According to an embodiment, the data satisfying the specified condition may include data that is selected based on the type of data and the measurement period included in the specified condition, from among data measured through at least one sensor that is selected based on the type of a sensor included in the specified condition from among a plurality of sensors included in the external electronic device.

In operation 470, the processor may display at least one object that is provided based on the received data, through a display. For example, the processor may provide at least one object, based on the data received from the external electronic device via the communication circuit, and may display the provided at least one object through the display. The at least one object may include, for example, information related to a user's health state. The information related to the user's health state may include, for example, at least one of user's biometric information, user's activity information, or health information that is determined based on the user's biometric information and activity information. The at least one object may include at least one of a text and an image. In some embodiment, the processor may output voice information corresponding to the at least one object, through a speaker (e.g., the sound output module 155 of FIG 1).

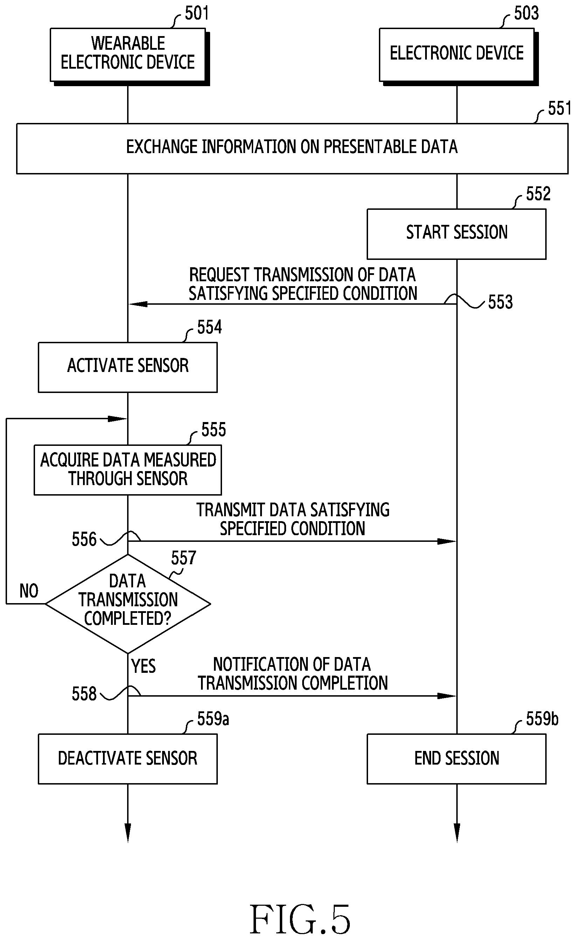

FIG 5 is a diagram for explaining a method of transmitting data through a session connection between a wearable electronic device and an electronic device according to an embodiment of the present invention.

Referring to FIG 5, the wearable electronic device 501 (e.g., the wearable electronic device 201 of FIG 2) and the electronic device 503 (e.g., the electronic device 203 of FIG 2) may transmit and/or receive data through a session. The session may represent a logical connection including a period from the start to the end of communication for transmission and/or reception of data, in communication connection between devices (e.g., the wearable electronic device 501 and the electronic device 503) via respective communication circuits (e.g., the communication circuit 213 and the communication circuit 231 of FIG 2). When the session is established, the wearable electronic device 501 and the electronic device 503 may transmit and/or receive data satisfying a specified condition while the established session is maintained. Since one session requires only one data transmission request, a plurality of data satisfying the specified condition may be transmitted and/or received while the one session is maintained.

In operation 551, the wearable electronic device 501 and the electronic device 503 may exchange information on data that may be presented to each other. For example, the wearable electronic device 501 may transmit information on data measurable through a sensor (e.g., the sensor 211 of FIG 2) included in the wearable electronic device 501, to the electronic device 503 via a communication circuit (e.g., the communication circuit 213 of FIG 2) included in the wearable electronic device 501. The information on the data measurable through the sensor may include, for example, information on the type of the sensor included in the wearable electronic device 501 and the type of the data measurable through the sensor. Also, the electronic device 503 may transmit at least one of information on data measurable through a sensor (e.g., the sensor 239 of FIG 2) included in the electronic device 503 or information on data stored in a memory (e.g., the memory 235 of FIG 2), to the wearable electronic device 501 via a communication circuit (e.g., the communication circuit 231 of FIG 2) included in the electronic device 503. In operation 551, it has been described that the wearable electronic device 501 and the electronic device 503 exchange information with each other, but in some embodiment, only the operation of the wearable electronic device 501 transmitting information to the electronic device 503 may be performed as well.

In operation 552, a session between the wearable electronic device 501 and the electronic device 503 may be started. For example, the electronic device 503 may establish a session for data transmission and/or reception, and may control the established session to start. In operation 552, it has been described that the session is established and started by the electronic device 503 that is a subject of a data transmission request, but in some embodiment, in operation 553, the session may be established and started by the wearable electronic device 501 receiving a data transmission request as well.

In operation 553, the electronic device 503 may request the transmission of data satisfying a specified condition, to the wearable electronic device 501. For example, the electronic device 503 may request the transmission of data satisfying a specified condition, to the wearable electronic device 501 through the session. In some embodiment, when the session is established by the wearable electronic device 501, the data transmission request satisfying the specified condition may be transmitted separately from the session.

The specified condition may be set based on information on the type of a sensor included in the wearable electronic device 501 and the type of data measurable through the sensor. According to an embodiment, the electronic device 503 may display, through a display (e.g., the display 233 of FIG 2), the information on the type of the sensor included in the wearable electronic device 501 received from the wearable electronic device 501 and the type of the data measurable through the sensor. For example, the electronic device 503 may display the information on the type of the sensor included in the wearable electronic device 501 and the type of the data measurable through the sensor, on the display, in the form of a list, and may set the specified condition, based on a user input for selecting at least one of items included in the list. Here, each of the items included in the list may include an object to which information on the type of the sensor included in the wearable electronic device 501, the type of the data measurable through the sensor, and the measurement period is mapped.

In operation 554, the wearable electronic device 501 receiving the transmission request may activate the sensor included in the wearable electronic device 501. For example, the wearable electronic device 501 may activate the sensor included in the wearable electronic device 501, based on the type of the sensor included in the specified condition. According to an embodiment, when the wearable electronic device 501 includes a plurality of sensors, the wearable electronic device 501 may select at least one sensor among the plurality of sensors, based on the type of the sensor included in the specified condition, and may activate the selected at least one sensor.

In operation 555, the wearable electronic device 501 may acquire data measured through the activated sensor. For example, the wearable electronic device 501 may acquire data related to at least one of user's biometric information and activity information through the activated sensor. The user's biometric information may include, for example, at least one of a user's heart rate, blood pressure, blood sugar, blood volume, oxygen saturation, and electrocardiogram. The user's activity information may include, for example, at least one of information (e.g., the number of steps) on an exercise state such as running or walking, or information on a sleep state such as the degree of movement during sleep or a sleeping posture.

When the data is acquired through the activated sensor, the wearable electronic device 501 may select data satisfying the specified condition from among the data acquired through the activated sensor. For example, the wearable electronic device 501 may select data corresponding to the type of data and the measurement period included in the specified condition, from among the data acquired through the activated sensor.

According to an embodiment, when the data requested to be transmitted (operation 553) includes data previously already measured by a sensor included in the wearable electronic device 510 with a criterion of a current time point, the wearable electronic device 501 may not perform operation 554, operation 555, and operation 559a. For example, the wearable electronic device 501 may omit the activation/deactivation of the sensor and the acquisition of data through the sensor. In this case, the wearable electronic device 501 may use data that has already been measured through the sensor and stored in the memory (e.g., the memory 217 of FIG 2). In this regard, the wearable electronic device 501 may determine whether the data requested to be transmitted includes the data previously already measured through the sensor with a criterion of the current time point, based on at least one of the sensor type, the data type, and the measurement period.

In operation 556, the wearable electronic device 501 may transmit the selected data to the electronic device 503 via the communication circuit. For example, the wearable electronic device 501 may transmit data satisfying the specified condition among the acquired data, to the electronic device 503 via the communication circuit.

In operation 557, the wearable electronic device 501 may determine whether the transmission of the data satisfying the specified condition has been completed. For example, the wearable electronic device 501 may determine whether all the data satisfying the specified condition among data measurable through the activated sensor have been transmitted. When it is determined that the transmission of the data satisfying the specified condition has not been completed (that is, when it is determined that the data satisfying the specified condition remains), the wearable electronic device 501 may continuously perform operation 555 and operation 556. That is, until data satisfying the specified condition does not remain, the wearable electronic device 501 may continuously perform the transmission of the data.

When it is determined that the transmission of data satisfying the specified condition has been completed (that is, when it is determined that the data satisfying the specified condition does not remain), in operation 558, the wearable electronic device 501 may transmit a data transmission completion notification to the electronic device 503 via the communication circuit. In some embodiment, the electronic device 503 may determine that it has received all the data of the specified condition without receiving the data transmission completion notification from the wearable electronic device 501 as well. In an example, the electronic device 503 may determine the data transmission completion, based on the measurement period of the sensor included in the specified condition. For example, when the measurement period of the sensor included in the specified condition exceeds a current time, the electronic device 503 may determine the data transmission completion.

When the data transmission is completed, in operation 559a, the wearable electronic device 501 may deactivate the activated sensor. Also, when the data transmission is completed, in operation 559b, the electronic device 503 may end the session. In some embodiment, when the session is established and started by the wearable electronic device 501, the end of the session may also be performed by the wearable electronic device 501. For example, in operation 559a, the wearable electronic device 501 may end the session.

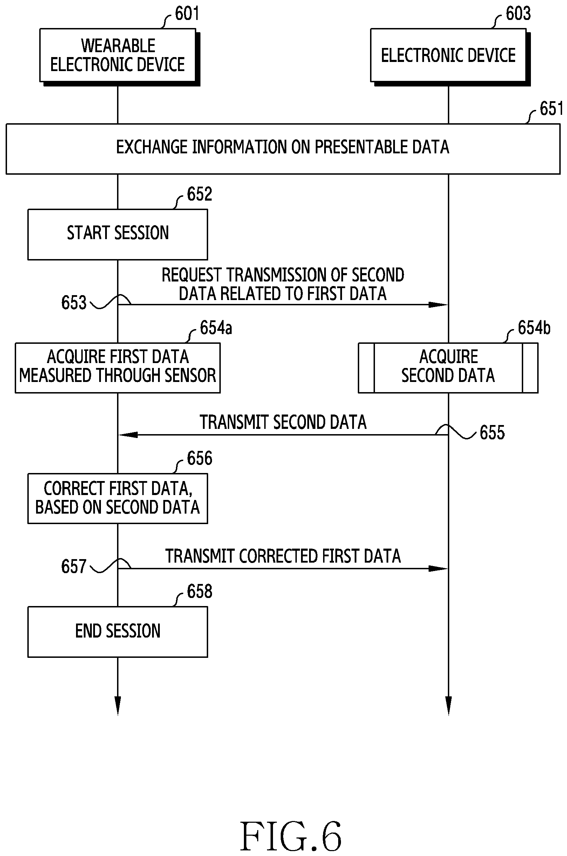

FIG 6 is a diagram for explaining another method of transmitting data through a session connection between a wearable electronic device and an electronic device according to an embodiment of the present invention.

Referring to FIG 6, the wearable electronic device 601 (e.g., the wearable electronic device 201 of FIG 2) and the electronic device 603 (e.g., the electronic device 203 of FIG 2) may transmit and/or receive data through a session. In operation 651, the wearable electronic device 601 and the electronic device 603 may exchange information on data that may be presented to each other. For example, the wearable electronic device 601 may transmit information on data measurable through a sensor (e.g., the sensor 211 of FIG 2) included in the wearable electronic device 601, to the electronic device 603 via a communication circuit (e.g., the communication circuit 213 of FIG 2) included in the wearable electronic device 601. The information on the data measurable through the sensor may include, for example, information on the type of the sensor included in the wearable electronic device 601 and the type of the data measurable through the sensor. Also, the electronic device 603 may transmit at least one of information on data measurable through a sensor (e.g., the sensor 239 of FIG 2) included in the electronic device 603 or information on data stored in a memory (e.g., the memory 235 of FIG 2), to the wearable electronic device 601 via a communication circuit (e.g., the communication circuit 231 of FIG 2) included in the electronic device 603. In operation 651, it has been described that the wearable electronic device 601 and the electronic device 603 exchange information with each other, but in some embodiment, only an operation of the electronic device 603 transmitting the information to the wearable electronic device 601 may be performed as well.

In operation 652, a session between the wearable electronic device 601 and the electronic device 603 may be started. For example, the wearable electronic device 601 may establish a session for data transmission and/or reception, and control the established session to start. In operation 652, it has been described that the session is established and started by the wearable electronic device 601 that is a subject of a request for transmission of second data, but in some embodiment, in operation 653, the session may be established and started by the electronic device 603 having received the request for transmission of the second data. Or, as described in operation 552 of FIG 5, the session is established and started by the electronic device 603 that is a subject of a request for transmission of first data, and a request for transmission of second data related to the first data may be transmitted through the already established session as well. In this case, the end of the session may also be performed by the electronic device 603 as in operation 559b of FIG 5.

In operation 653, the wearable electronic device 601 may request the transmission of second data related to the first data, to the electronic device 603 via the communication circuit. According to an embodiment, the first data may be data related to data requested to be transmitted from the electronic device 603 (e.g., data requested to be transmitted in FIG 5), and the second data may be data used for measurement or detection of the first data. In an example, when the first data is data indicating a user's calorie consumption amount, the second data may be data indicating at least one of a user's gender, height, weight, stride length, or an altitude value of a user's current location. In another example, when the first data is data indicating a user's sleep state or stress state, the second data may be data indicating a user's caffeine intake amount or exercise amount before sleep. In a further example, when the first data is data indicating a heart disease state being based on a user's blood sugar or electrocardiogram, the second data may be data indicating user's food intake information.

After requesting the transmission of the second data, in operation 654a, the wearable electronic device 601 may acquire first data measured through a sensor included in the wearable electronic device 601.

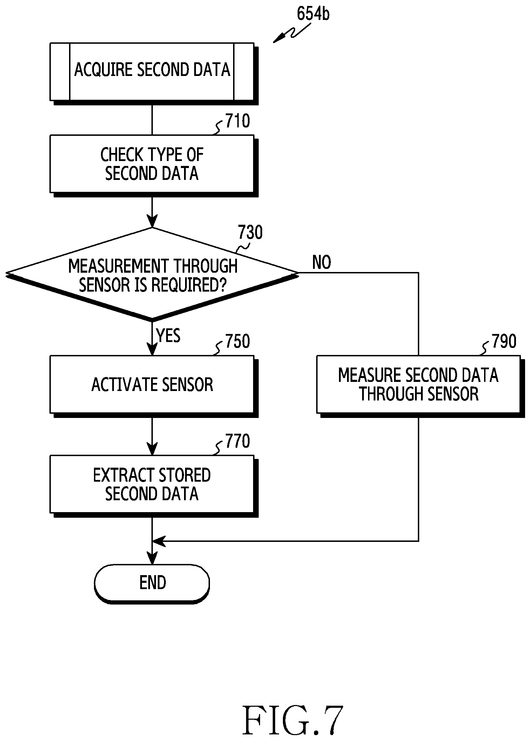

In operation 654b, the electronic device 603 having received the request for transmission of the second data may acquire the second data. Operations related to the acquisition of the second data will be described in detail with reference to FIG 7.

In operation 655, the electronic device 603 having acquired the second data may transmit the acquired second data to the wearable electronic device 601 via the communication circuit. According to an embodiment, an operation (operation 654a) of the wearable electronic device 601 acquiring the first data and an operation (operation 654b) of the electronic device 603 acquiring the second data may be simultaneously performed. In another embodiment, the operation (operation 654a) of the wearable electronic device 601 acquiring the first data may be performed before the operation (operation 654b) of the electronic device 603 acquiring the second data. In a further embodiment, the operation (operation 654a) of the wearable electronic device 601 acquiring the first data may be performed later than the operation (operation 654b) of the electronic device 603 acquiring the second data and an operation (operation 655) of the electronic device 603 transmitting the second data to the wearable electronic device 601 as well. When operation 654a is performed later than operations 654b and 655, in operation 654a, the wearable electronic device 601 may selectively acquire only first data satisfying a specified condition that is set based on acquired second data, among data measured through a sensor as well. In this case, the operation of correcting the first data in operation 656 may be omitted.

In operation 656, the wearable electronic device 601 having received the second data may correct the first data acquired through the sensor, based on the received second data. In an example, the wearable electronic device 601 may present the first data indicating a user's calorie consumption amount, based on the second data indicating at least one of a user's gender, height, weight, stride length, or an altitude value of a user's current location. In another example, the wearable electronic device 601 may correct the first data indicating a user's sleep state or stress state, based on the second data indicating a user's caffeine intake amount or exercise amount before sleep. In a further example, the wearable electronic device 601 may correct the first data indicating a heart disease state being based on a user's blood sugar or electrocardiogram, based on the second data indicating user's food intake information. Accordingly, since the wearable electronic device 601 may more accurately reflect a user's physical condition or a user's current state at the time of measuring or detecting the first data, the wearable electronic device 601 may improve the accuracy and reliability of the first data.

In operation 657, the wearable electronic device 601 may transmit the corrected first data to the electronic device 603 via the communication circuit. Also, in operation 658, the wearable electronic device 601 may end the session. In some embodiment, when the session is established and started by the electronic device 603, the end of the session may also be performed by the electronic device 603.

FIG 7 is a diagram for explaining a method of acquiring necessary data according to the type of data according to an embodiment of the present invention. Operations illustrated in FIG 7 may represent detailed operations for an operation (operation 654b) of acquiring the second data in FIG 6.

Referring to FIG 7, the electronic device 603 receiving a request for transmission of second data related to first data may acquire the second data. In operation 710, the electronic device 603 may check the type of the second data of which the transmission request has been received. The second data may include data measurable through a sensor (e.g., the sensor 239 of FIG 2) included in the electronic device 603 or data stored in a memory (e.g., the memory 235 of FIG 2) of the electronic device 603.

In operation 730, the electronic device 603 may determine whether the measurement of the second data through the sensor included in the electronic device 603 is required. For example, the electronic device 603 may determine whether the type of the second data checked in operation 710 is data measurable through the sensor.

When the second data is required to be measured through the sensor (i.e., when the second data is the data measurable through the sensor), in operation 750, the electronic device 603 may activate the sensor (e.g., the sensor 239 of FIG 2) for measuring the second data. Also, in operation 770, the electronic device 603 may acquire the second data, by measuring the second data through the activated sensor. The second data measured through the activated sensor may include, for example, at least one of a user's exercise amount or an altitude value of a user's current location. In this case, the activated sensor may include, for example, at least one of an acceleration sensor, a gyro sensor, and a geomagnetic sensor for measuring the exercise amount, and may include at least one of a GPS sensor or an altimeter for measuring an altitude value.

When the second data is not required to be measured through the sensor (i.e., when the second data is not the data measurable through the sensor), in operation 790, the electronic device 603 may acquire the second data by extracting the second data from data stored in a memory (e.g., the memory 235 of FIG 2). The data stored in the memory may include, for example, at least one of user's profile information, a user's caffeine intake amount before sleep, and user's food intake information. The user's profile information may include, for example, information on at least one of a user's gender, height, and weight. The user's caffeine intake amount or food intake information may be stored in the memory, based on a user input. In some embodiment, the second data may include data on a user's stride length that is calculated based on a user's height stored in the memory.

According to an embodiment, the data stored in the memory may be stored and managed in the form of a table. In this case, the electronic device 603 may extract only the second data from the table. In some embodiment, when the data stored in the memory is stored in a plurality of tables and the second data is stored separately in the plurality of tables, the electronic device 603 may extract all of the second data from the plurality of tables. Also, the electronic device 603 may merge the second data extracted from each of the plurality of tables as well.

As described above, according to various embodiments, a data transmission method may include an operation (e.g., operation 310 of FIG 3) of receiving a request for transmission of first data satisfying a specified condition among data measurable using at least one sensor (e.g., the sensor 211 of FIG 2), from an external electronic device (e.g., the electronic device 203 of FIG 2) connected via a communication circuit (e.g., the communication circuit 213 of FIG 2), an operation (e.g., operation 330 of FIG 3) of acquiring the data measured through the at least one sensor, and an operation (e.g., operation 350 of FIG 3) of transmitting the first data satisfying the specified condition among the acquired data, to the external electronic device via the communication circuit.

According to various embodiments, the data transmission method may further include an operation of establishing a first session for transmission of the first data, in response to the reception of the request for transmission of the first data.

According to various embodiments, the data transmission method may further include an operation of, when receiving a request for transmission of second data satisfying a condition different from the specified condition among the data from the external electronic device via the communication circuit, establishing a second session for the transmission of the second data.

According to various embodiments, the data transmission method may include an operation of transmitting information on data measurable using the at least one sensor to the external electronic device via the communication circuit (e.g., operation 551 of FIG 5 or operation 651 of FIG 6).

According to various embodiments, the data transmission method may further include an operation (e.g., operation 653 of FIG 6) of transmitting a request for transmission of second data related to the first data to the external electronic device via the communication circuit, and an operation (e.g., operation 656 of FIG 6) of, when receiving the second data from the external electronic device via the communication circuit, correcting the first data, based on the received second data, and an operation of transmitting the first data may include an operation (e.g., operation 657 of FIG 6) of transmitting the corrected first data to the external electronic device via the communication circuit.

According to various embodiments, the data transmission method may further include an operation of transmitting a plurality of second data measured by the at least one sensor and a request for processing of the plurality of second data to the external electronic device via the communication circuit, and an operation of receiving third data calculated by processing the plurality of second data from the external electronic device via the communication circuit.

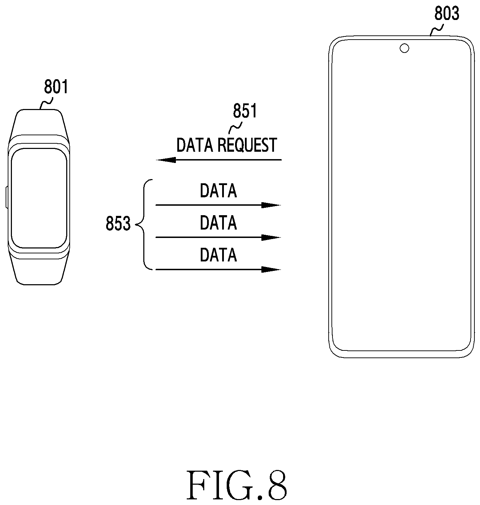

FIG 8 is a diagram for explaining a process of data transmission between a wearable electronic device and an electronic device according to an embodiment of the present invention.

Referring to FIG 8, the electronic device 803 (e.g., the electronic device 203 of FIG 2) may receive data related to user's biometric information or user's activity information measured through a sensor (e.g., the sensor 211 of FIG 2) included in the wearable electronic device 801, from the wearable electronic device 801 (e.g., the wearable electronic device 201 of FIG 2) connected via a communication circuit (e.g., the communication circuit 231 of FIG 2). Also, the electronic device 803 may determine a user's health state, based on data received from the wearable electronic device 801, and may display information related to the determined health state through a display (e.g., the display 233 of FIG 2). In this case, the electronic device 803 may selectively or variably receive only necessary data, for example, data related to a health state requested by a user, without receiving all the data measured through the sensor included in the wearable electronic device 801. Accordingly, the consumption of communication resources for data transmission and/or reception between the wearable electronic device 801 and the electronic device 803 may be reduced, and the consumption of resources required for data processing, for example, a memory (e.g., the memory 217 and the memory 235), a CPU (e.g., the processor 215 and the processor 237), or a battery may be reduced.

To perform the above-described function, in operation 851, the electronic device 803 may request the necessary data to the wearable electronic device 801 via the communication circuit. For example, the electronic device 803 may transmit a data transmission request satisfying a specified condition to the wearable electronic device 801 via the communication circuit. The specified condition may include, for example, the type of a sensor used for measurement among sensors included in the wearable electronic device 801, the type of data measured through the selected sensor, and a measurement period. The sensor used for the measurement may represent a sensor used to acquire the necessary data, and the type of data and the measurement period may represent the type of the necessary data among the data measured through the sensor, and a condition of the necessary data.

The wearable electronic device 801 receiving the data transmission request satisfying the specified condition may acquire data (sensing data) through a sensor that is selected based on the type of the sensor included in the specified condition. According to an embodiment, the wearable electronic device 801 may activate the sensor that is selected based on the type of the sensor included in the specified condition, and may acquire data through the activated sensor.

When the data is acquired through the sensor, in operation 853, the wearable electronic device 801 may transmit data satisfying the specified condition among the acquired data, to the electronic device 803 via a communication circuit (e.g., the communication circuit 213 of FIG 2). For example, the wearable electronic device 801 may select data corresponding to the type of data and the measurement period included in the specified condition, from among the acquired data, and transmit the selected data to the electronic device 803 via the communication circuit. In this case, until all data satisfying a specified condition are transmitted, the wearable electronic device 801 may repeatedly perform an operation of acquiring data through the sensor and an operation of selecting data satisfying the specified condition from among the acquired data. For example, the wearable electronic device 801 may transmit a plurality of data satisfying the specified condition, to the electronic device 803 via the communication circuit. According to an embodiment, the wearable electronic device 801 may deactivate the activated sensor after transmitting all the data satisfying the specified condition to the electronic device 803.

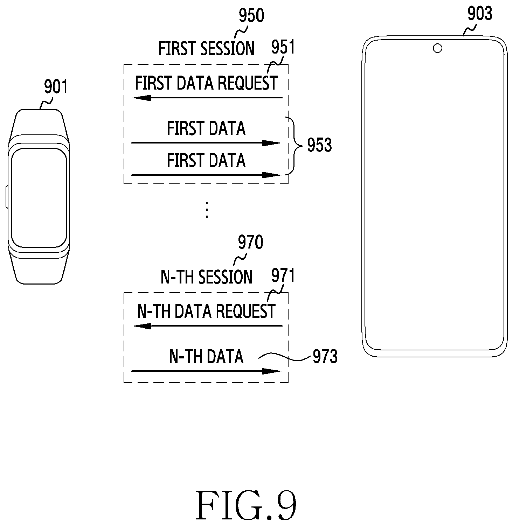

FIG 9 is a diagram for explaining a process of data transmission through a session connection between a wearable electronic device and an electronic device according to an embodiment of the present invention.

According to an embodiment, the wearable electronic device 901 (e.g., the wearable electronic device 201 of FIG 2) and the electronic device 903 (e.g., the electronic device 203 of FIG 2) may transmit and/or receive data through a session. For example, the operation (operation 851) of requesting the transmission of data satisfying the specified condition and the operation (operation 853) of transmitting the plurality of data satisfying the specified condition described in FIG 8 may be performed using one session. The session may indicate a logical connection that includes a period from the start to the end of communication for transmission and/or reception of data, in a communication connection between devices (e.g., the wearable electronic device 901 and the electronic device 903) via respective communication circuits (e.g., the communication circuit 213 and the communication circuit 231 of FIG 2). In FIG 9, a method of transmitting and/or receiving a plurality of data satisfying different conditions through a plurality of sessions will be described.

Referring to FIG 9, when a plurality of data satisfying different conditions are transmitted and/or received between the wearable electronic device 901 and the electronic device 903, a plurality of different sessions may be established to transmit and/or receive data satisfying each condition. For example, the wearable electronic device 901 and the electronic device 903 may transmit and/or receive data satisfying a first specified condition through a first session 950, and may transmit and/or receive data satisfying an n-th specified condition through an n-th session 970. Here, the n is a natural number, and indicates that the number of sessions and the number of specified conditions are plural.

In operation 951, the electronic device 903 may request first data satisfying a first specified condition to the wearable electronic device 901 via the communication circuit. For example, the electronic device 903 may transmit a request for transmission of the first data satisfying the first specified condition, to the wearable electronic device 901 via the communication circuit. In this case, the electronic device 903 may establish the first session 950, and may transmit the request for transmission of the first data satisfying the first specified condition through the established first session 950.

The wearable electronic device 901 having received the request for transmission of the first data satisfying the first specified condition may acquire data (first sensing data) through a first sensor (e.g., the sensor 211 of FIG 2) that is selected based on the type of a sensor included in the first specified condition. According to an embodiment, the wearable electronic device 901 may activate the first sensor that is selected based on the type of the sensor included in the first specified condition, and may acquire the first sensing data through the activated first sensor.

When the first sensing data is acquired through the first sensor, in operation 953, the wearable electronic device 901 may transmit the first data satisfying the first specified condition among the acquired first sensing data, to the electronic device 903 via the communication circuit. For example, the wearable electronic device 901 may select first data corresponding to the type of data included in the first specified condition and a measurement period from among the acquired first sensing data, and may transmit the selected first data to the electronic device 903 via the communication circuit. In this case, the wearable electronic device 901 may transmit the first data to the electronic device 903 through the first session 950 and, until all the first data satisfying the first specified condition are transmitted, the wearable electronic device 901 may repeatedly perform an operation of acquiring the first sensing data through the first sensor and an operation of selecting the first data satisfying the first specified condition from among the acquired first sensing data. According to an embodiment, the wearable electronic device 901 may deactivate the activated first sensor, after transmitting all the first data satisfying the first specified condition to the electronic device 903. Also, the electronic device 903 having received all the first data satisfying the first specified condition from the wearable electronic device 901 may end the first session 950.

To acquire data satisfying another condition, the electronic device 903 may perform a data transmission request operation and a data reception operation through a session different from the first session 950. For example, as in operation 971, the electronic device 903 may request n-th data satisfying an n-th specified condition to the wearable electronic device 901 via the communication circuit. In this case, the electronic device 903 may establish an n-th session 970, and may transmit a request for transmission of the n-th data satisfying the n-th specified condition through the set n-th session 970. The wearable electronic device 901 having received the request for transmission of the n-th data may activate an n-th sensor (e.g., the sensor 211 of FIG 2) that is selected based on the type of a sensor included in the n-th specified condition, and may acquire data (n-th sensing data) through the activated n-th sensor. Also, when the n-th sensing data is acquired through the n-th sensor, in operation 973, the wearable electronic device 901 may transmit the n-th data satisfying the n-th specified condition among the acquired n-th sensing data, to the electronic device 903 via the communication circuit. For example, the wearable electronic device 901 may select the n-th data corresponding to the type of data included in the n-th specified condition and the measurement period from among the acquired n-th sensing data, and may transmit the selected n-th data to the electronic device 903 via the communication circuit. In this case, the wearable electronic device 901 may transmit the n-th data to the electronic device 903 through the n-th session 970. Thereafter, the wearable electronic device 901 may deactivate the activated n-th sensor. Also, the electronic device 903 having received all the n-th data satisfying the n-th specified condition from the wearable electronic device 901 may end the n-th session 970.

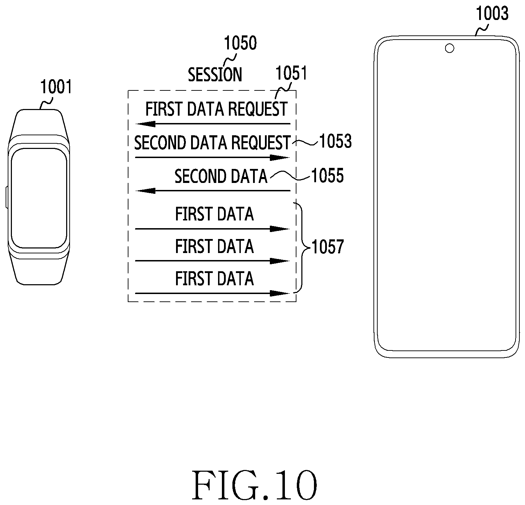

FIG 10 is a diagram for explaining another process of data transmission through a session connection between a wearable electronic device and an electronic device according to an embodiment of the present invention.

Referring to FIG 10, the wearable electronic device 1001 (e.g., the wearable electronic device 201 of FIG 2) and the electronic device 1003 (e.g., the electronic device 203 of FIG 2) may transmit and/or receive data through a session. In this case, in order to increase the accuracy and reliability of data (first data) transmitted to the electronic device 1003, the wearable electronic device 1001 may request data (second data) including additional information to the electronic device 1003, and may correct and transmit data to be transmitted to the electronic device 1003, based on data received from the electronic device 1003.

In operation 1051, the electronic device 1003 may transmit a request for transmission of first data satisfying a specified condition, to the wearable electronic device 1001 via a communication circuit (e.g., the communication circuit 231 of FIG 2). In this case, the electronic device 1003 may establish a session 1050, and may transmit the request for transmission of the first data satisfying the specified condition through the established session 1050.

In operation 1053, the wearable electronic device 1001 having received the request for transmission of the first data satisfying the specified condition may transmit a request for transmission of second data related to the first data, to the electronic device 1003 via a communication circuit (e.g., the communication circuit 213 of FIG 2). In this case, the wearable electronic device 1001 may transmit the request for transmission of the second data through the session 1050.

According to an embodiment, the second data may be data used to measure or detect the first data. In an example, when the first data is data indicating a user's calorie consumption amount, the second data may be data indicating at least one of a user's gender, height, weight, stride length, or an altitude value of a user's current location. In another example, when the first data is data indicating a user's sleep state or stress state, the second data may be data indicating a user's caffeine intake amount or exercise amount before sleep. In a further example, when the first data is data indicating a heart disease state being based on a user's blood sugar or electrocardiogram, the second data may be data indicating user's food intake information.

The electronic device 1003 having received the request for transmission of the second data may acquire the second data by using a sensor (e.g., the sensor 239 of FIG 2) included in the electronic device 1003 or may acquire the second data from data stored in a memory (e.g., the memory 235 of FIG 2) included in the electronic device 1003 in accordance with the type of the second data. Since the operation of acquiring the second data may be the same as or be similar to the operations (operations 710 to 790) described with reference to FIG 7, a detailed description will be omitted.

In operation 1055, the electronic device 1003 having acquired the second data may transmit the acquired second data to the wearable electronic device 1001 via the communication circuit. In this case, the electronic device 1003 may transmit the acquired second data through the session 1050.

Meanwhile, the wearable electronic device 1001 having received the request for transmission of the first data may acquire sensing data through a sensor (e.g., the sensor 211 of FIG 2) that is selected based on the type of the sensor included in the specified condition. According to an embodiment, the wearable electronic device 1001 may activate the sensor that is selected based on the type of the sensor included in the specified condition, and may acquire sensing data through the activated sensor. Also, the wearable electronic device 1001 may select the first data satisfying the specified condition from among the sensing data acquired through the sensor. For example, the wearable electronic device 1001 may select the first data corresponding to the type of data and the measurement period included in the specified condition, from among the acquired sensing data.

The wearable electronic device 1001 having received the second data may correct the first data acquired through the sensor, based on the second data. In an example, the wearable electronic device 1001 may correct the first data indicating a user's calorie consumption amount, based on the second data indicating at least one of a user's gender, height, weight, stride length, or an altitude value of a user's current location. In another example, the wearable electronic device 1001 may correct the first data indicating a user's sleep state or stress state, based on the second data indicating a user's caffeine intake amount or exercise amount before sleep. In a further example, the wearable electronic device 1001 may correct the first data indicating a heart disease state being based on a user's blood sugar or electrocardiogram, based on the second data indicating user's food intake information.

After correcting the first data, in operation 1057, the wearable electronic device 1001 may transmit the corrected first data to the electronic device 1003 via the communication circuit. In this case, the wearable electronic device 1001 may transmit the first data to the electronic device 1003 through the session 1050, and until all the corrected first data satisfying the specified condition are transmitted, the wearable electronic device 1001 may repeatedly perform an operation of acquiring sensing data through the sensor, an operation of selecting the first data satisfying the specified condition among the acquired sensing data, and an operation of correcting the first data, based on the second data. According to an embodiment, the wearable electronic device 1001 may deactivate the activated sensor, after transmitting all the first data satisfying the specified condition to the electronic device 1003. Also, the electronic device 1003 having received all the first data satisfying the specified condition from the wearable electronic device 1001 may end the session 1050.

According to an embodiment, an operation of the wearable electronic device 1001 acquiring the first data and an operation of the electronic device 1003 acquiring the second data may be simultaneously performed. In another embodiment, the operation of the wearable electronic device 1001 acquiring the first data may be performed before the operation of the electronic device 1003 acquiring the second data. In a further embodiment, the operation of the wearable electronic device 1001 acquiring the first data may be performed later than the operation of the electronic device 1003 acquiring the second data and the operation of the electronic device 1003 transmitting the second data to the wearable electronic device 1001 as well. In this case, the wearable electronic device 1001 may selectively acquire only the first data satisfying the specified condition that is set based on the acquired second data, among the sensing data measured through the sensor as well. Also, in this case, the operation of correcting the first data may be omitted.

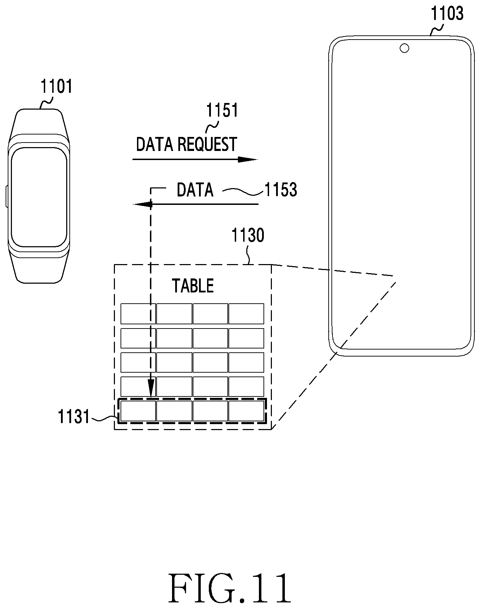

FIG 11 is a diagram for explaining a process of acquiring necessary data according to the type of data according to an embodiment of the present invention.

Referring to FIG 11, in operation 1151, the wearable electronic device 1101 (e.g., the wearable electronic device 201 of FIG 2) may request data to the electronic device 1103 (e.g., the electronic device 203 of FIG 2) via a communication circuit (e.g., the communication circuit 213 of FIG 2). According to an embodiment, the data (e.g., the second data of FIG 10) requested by the wearable electronic device 1101 to the electronic device 1103 may be data used by the wearable electronic device 1101 to increase the accuracy and reliability of data (e.g., the first data of FIG 10) requested to be transmitted from the electronic device 1103.

The electronic device 1103 having received the data transmission request may acquire the requested data by using a sensor (e.g., the sensor 239 of FIG 2) included in the electronic device 1103 or may acquire the data by extracting the requested data from data stored in a memory (e.g., the memory 235 of FIG 2) included in the electronic device 1103, in accordance with the type of the requested data. In FIG 11, a method in which the electronic device 1103 acquires the requested data from the memory will be described.

The electronic device 1103 may extract the requested data from among the data stored in the memory. The data stored in the memory may include, for example, at least one of user's profile information, a user's caffeine intake amount before sleep, or user's food intake information. The user's profile information may include, for example, information on at least one of a user's gender, height, and weight. The user's caffeine intake amount or food intake information may be stored in the memory, based on a user input. In some embodiment, the requested data may include data on a user's stride length that is calculated based on the user's height stored in the memory.

According to an embodiment, the data stored in the memory may be stored and managed in the form of a table 1130. In this case, the electronic device 1103 may extract only the requested data 1131 from the table 1130.

In operation 1153, the electronic device 1103 acquiring the requested data from the memory may transmit the acquired data (requested data) to the wearable electronic device 1101 via a communication circuit (e.g., the communication circuit 231 of FIG 2). According to an embodiment, the data request operation (operation 1151) and the requested data transmission operation (operation 1153) may be performed through a session.

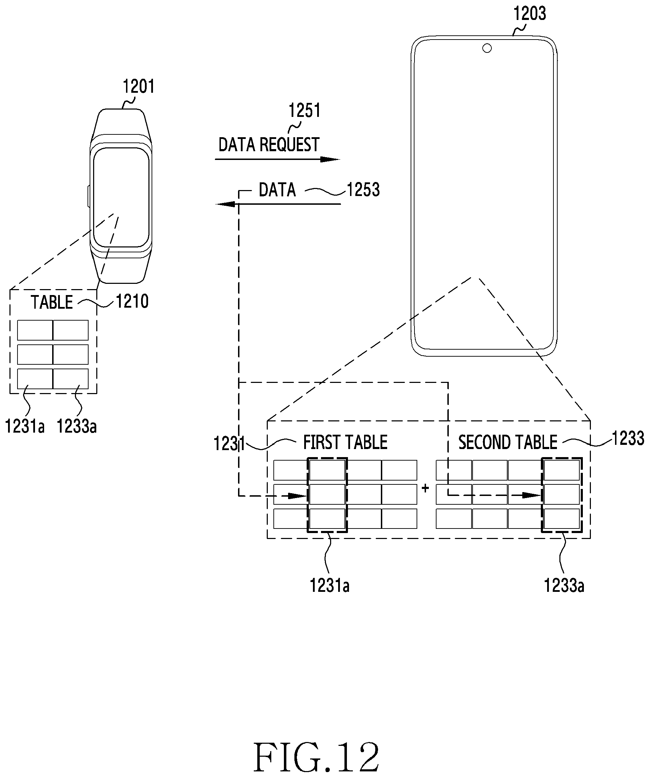

FIG 12 is a diagram for explaining another acquisition process of necessary data according to the type of data according to an embodiment of the present invention. The data acquisition operations of FIG 12 may be similar to the data acquisition operations of FIG 11. Accordingly, a description of operations similar to those of FIG 11 will be omitted.

Referring to FIG 12, in operation 1251, the wearable electronic device 1201 (e.g., the wearable electronic device 201 of FIG 2) may request data to the electronic device 1203 (e.g., the electronic device 203 of FIG 2) via a communication circuit (e.g., the communication circuit 213 of FIG 2).

The electronic device 1203 may extract the requested data from data stored in a memory (e.g., the memory 235 of FIG 2). According to an embodiment, the data stored in the memory may be stored and managed in the form of a table (e.g., a first table 1231 and a second table 1233), and a plurality of tables may be stored in the memory.

When the requested data is stored separately in a plurality of tables, the electronic device 1203 may extract the requested data from each of the plurality of tables. For example, the electronic device 1203 may extract a first portion 1231a of the requested data from the first table 1231 stored in the memory, and may extract a second portion 1233a of the requested data from the second table 1233 stored in the memory.

In operation 1253, the electronic device 1203 having acquired the requested data from the memory may transmit the acquired data (requested data) to the wearable electronic device 1201 via a communication circuit (e.g., the communication circuit 231 of FIG 2). According to an embodiment, the electronic device 1203 may merge portions of the requested data extracted from each of the plurality of tables and transmit the merged portions to the wearable electronic device 1201. For example, the electronic device 1203 may merge the first portion 1231a extracted from the first table 1231 and the second portion 1233a extracted from the second table 1233 and transmit to the wearable electronic device 1201. According to an embodiment, the data request operation (operation 1251) and the requested data transmission operation (operation 1253) may be performed through a session.

The wearable electronic device 1201 having received the requested data from the electronic device 1203 may store the received data in a memory (e.g., the memory 217 of FIG 2). According to an embodiment, the wearable electronic device 1201 may store the received data in the memory in the form of a table 1210. For example, the wearable electronic device 1201 may store, in the memory, a table 1210 in which the first portion 1231a extracted from the first table 1231 and the second portion 1233a extracted from the second table 1233 are merged.

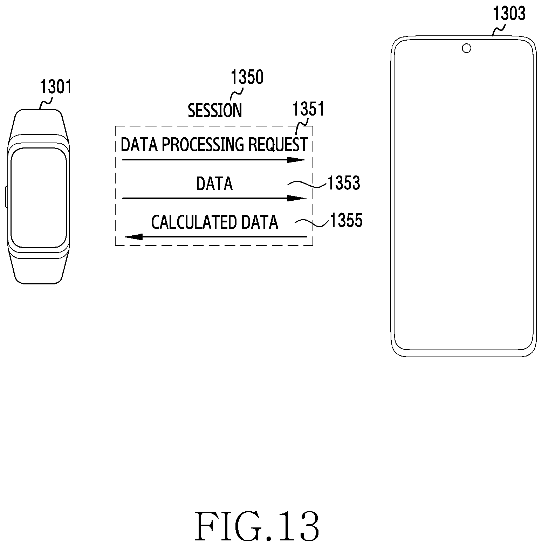

FIG 13 is a diagram for explaining a process of data processing request according to an embodiment of the present invention.

Referring to FIG 13, in a wearable electronic device 1301 (e.g., the wearable electronic device 201 of FIG 2), a storage space of a memory (e.g., the memory 217 of FIG 2) may be narrow due to device characteristics, and an arithmetic processing capability (e.g., an arithmetic processing speed and an arithmetic throughput) of a processor (e.g., the processor 215 of FIG 2) may be low. Accordingly, the wearable electronic device 1301 may request data processing to an electronic device 1303, which is relatively easier to secure the storage space of the memory and also has a high arithmetic processing capability of the processor, compared to the wearable electronic device 1301.

In operation 1351, the wearable electronic device 1301 may transmit a data processing request to the electronic device 1303 via a communication circuit. Also, in operation 1353, the wearable electronic device 1301 may transmit the data requested to be processed, to the electronic device 1303 via the communication circuit. According to an embodiment, an operation (operation 1351) of transmitting a data processing request and an operation (operation 1353) of transmitting data may be simultaneously performed. For example, the wearable electronic device 1301 may transmit the data processing request while transmitting the data to the electronic device 1303 via the communication circuit. The data may be, for example, data measured through a sensor (e.g., the sensor 211 of FIG 2) included in the wearable electronic device 1301.

According to an embodiment, the wearable electronic device 1301 may request the electronic device 1303 to calculate or analyze data, based on data measured through the sensor. In an example, when the wearable electronic device 1301 is possible to measure a heart rate through the sensor but has no analysis engine capable of analyzing a heart rate variability or arrhythmias, the wearable electronic device 1301 may request the analysis of the heart rate variability or arrhythmias while transmitting data on the measured heart rate to the electronic device 1303 via the communication circuit. In another example, when the wearable electronic device 1301 is possible to measure data indicating a sleep state through the sensor but has no analysis engine for four sleep stages, the wearable electronic device 1301 may request the analysis of the four sleep stages while transmitting the measured data indicating the sleep state to the electronic device 1303 via the communication circuit.

The electronic device 1303 having received the data and the data processing request may calculate or analyze data, based on the received data. Also, in operation 1355, the electronic device 1303 may transmit the calculated or analyzed data to the wearable electronic device 1301 via a communication circuit (e.g., the communication circuit 231 of FIG 2). According to an embodiment, the data processing request operation (operation 1351), the data transmission operation (operation 1353), and the calculated or analyzed data transmission operation (operation 1355) may be performed through a session 1350. The session 1350 may be established by the wearable electronic device 1301 requesting for data processing or may be established by the electronic device 1303 processing data. Also, the session 1350 may be ended when the operation (operation 1355) of transmitting the calculated or analyzed data is completed.

According to an embodiment, when requesting the processing of data measured through the sensor, the wearable electronic device 1301 may collect the data measured through the sensor in a bulk form and transmit it to the electronic device 1303. For example, the wearable electronic device 1301 may transmit a plurality of data of the same or similar type measured through the sensor, to the electronic device 1303. According to an embodiment, when the wearable electronic device 1301 requests the processing of data measured through the sensor, only data necessary for calculation and analysis among the data measured through the sensor may be selectively collected in a bulk form and be transmitted to the electronic device 1303.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}₎, or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device comprising:
at least one sensor;
a communication circuit; and
a processor functionally connected to the at least one sensor and the communication circuit,
wherein the processor is configured to:
receive a request for transmission of first data satisfying a specified condition among data that is measurable using the at least one sensor, from an external electronic device connected via the communication circuit;
acquire the data measured through the at least one sensor; and
transmit the first data satisfying the specified condition among the acquired data, to the external electronic device via the communication circuit.

2. The wearable electronic device of claim 1, wherein the processor is configured to establish a first session for transmission of the first data, in response to receiving the request for transmission of the first data.

3. The wearable electronic device of claim 2, wherein the processor is configured to establish a second session for transmission of second data, when receiving a request for transmission of the second data satisfying a condition different from the specified condition among the data from the external electronic device via the communication circuit.

4. The wearable electronic device of claim 1, wherein the processor is configured to transmit information on the data that is measurable using the at least one sensor, to the external electronic device via the communication circuit.

5. The wearable electronic device of claim 1, wherein the processor is configured to:
transmit a request for transmission of second data related to the first data, to the external electronic device via the communication circuit;
when receiving the second data from the external electronic device via the communication circuit, correcting the first data, based on the received second data; and
transmit the corrected first data to the external electronic device via the communication circuit.

6. The wearable electronic device of claim 1, wherein the processor is configured to:
transmit a plurality of second data measured by the at least one sensor and a request for processing of the plurality of second data, to the external electronic device via the communication circuit; and
receive third data that is calculated by processing the plurality of second data, from the external electronic device via the communication circuit.

7. An electronic device comprising:
a communication circuit;
a display;
a memory; and
a processor functionally connected to the communication circuit, the display and the memory,
wherein the processor is configured to:
receive information on data measurable through at least one sensor comprised in a wearable electronic device, from the wearable electronic device connected via the communication circuit;
transmit a request for transmission of first data satisfying a specified condition among the data, to the wearable electronic device via the communication circuit, based on the received information;
receive the first data from the wearable electronic device via the communication circuit; and
display, through the display, at least one first object that is provided based on the received first data.

8. The electronic device of claim 7, wherein the processor is configured to establish a first session for requesting the transmission of the first data and receiving the first data.

9. The electronic device of claim 8, wherein the processor is configured to establish a second session for requesting the transmission of second data satisfying a condition different from the specified condition among the data and receiving the second data.

10. The electronic device of claim 7, wherein the processor is configured to:
display, through the display, at least one second object that is provided based on the received information;
receive a user input for selecting the at least one second object; and
set the specified condition, based on the received user input.

11. The electronic device of claim 10, wherein the at least one second object comprises an object to which information on at least one of the type of the at least one sensor comprised in the wearable electronic device, the type of the data measurable through the at least one sensor, and a measurement period is mapped.

12. The electronic device of claim 7, wherein the processor is configured to:
when receiving a request for transmission of second data related to the first data from the wearable electronic device via the communication circuit, check the type of the second data;
acquire the second data by using at least one sensor comprised in the electronic device or acquire the second data through extraction from the memory, in accordance with the checked type of the second data; and
transmit the acquired second data to the wearable electronic device via the communication circuit.

13. The electronic device of claim 12, wherein the processor is configured to:
extract a first portion of the second data from a first table that is stored in the memory; and
extract a second portion of the second data from a second table that is stored in the memory.

14. The electronic device of claim 13, wherein the electronic device is configured to merge the extracted first portion of the second data and the extracted second portion of the second data and transmit the merged portion to the wearable electronic device via the communication circuit.

15. A data transmission method comprising:
receiving a request for transmission of first data satisfying a specified condition among data measurable using at least one sensor, from an external electronic device connected via a communication circuit;
acquiring the data measured through the at least one sensor; and
transmitting the first data satisfying the specified condition among the acquired data, to the external electronic device via the communication circuit.
